# EUROPEAN PATENT APPLICATION

(11) **EP 0 874 052 A2**
(43) Date of publication of application: **28.10.1998**
(21) Application number: 98107346.3
(22) Date of filing: 22.04.1998
(51) Int. Cl.: C12N 15/55, C12N 9/16, A61K 38/46, C07K 16/40, G01N 33/577

(54) **Nucleic acid encoding a human protein phosphatase**

(30) Priority: 22.04.1997 EP 97106658
(71) Applicant: BIOPHARM GESELLSCHAFT ZUR BIOTECHNOLOGISCHEN ENTWICKLUNG VON PHARMAKA mbH, 69115 Heidelberg (DE)
(72) Inventor: Hanke, Michael, Dr., 67454 Hassloch (DE); Paulista, Michael, 69181 Leimen (DE); Pohl, Jens, Dr., 76707 Hambrücken (DE)
(74) Representative: Müller-Boré & Partner Patentanwälte

(57) **Abstract**

The present invention relates to nucleic acids encoding a novel human protein phosphatase of the family of protein serine/threonine phosphatases. In particular, it relates to novel DNA sequences encoding serine/threonine protein phosphatase, to expression plasmids containing said nucleic acids, to host organisms transformed by said expression plasmids, to the production of said protein by culturing said transformant, to antibodies specifically binding to said phosphatase and to agonists and/or antagonists for said protein, and to antisense MP-19 nucleic acid. Furthermore, the invention relates to serine or threonine residues and epitopes comprising said residues dephosphorylated by said protein and pharmaceutical compositions comprising said protein or agonists or antagonists thereof for the treatment of diseases influenced by changes in phosphorylation which controls e.g. cell proliferation and/or differentiation, to diagnostic kits and to *in vitro* diagnostic methods for the detection of phosphorylation dependent diseases such as e.g. cancer.

SEQUENCE LISTING

(1) GENERAL INFORMATION:

(i) APPLICANT:
(A) NAME: Biopharm GmbH
(B) STREET: Czernyring 22
(C) CITY: Heidelberg
(E) COUNTRY: Germany
(F) POSTAL CODE (ZIP): 69115

(ii) TITLE OF INVENTION: Nucleic acid encoding a novel human protein
phosphatase

(iii) NUMBER OF SEQUENCES: 4

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(v) CURRENT APPLICATION DATA:
APPLICATION NUMBER: EP 98107346.3

(2) INFORMATION FOR SEQ ID NO: 1:

(i) SEQUENCE CHARACTERISTICS:
(A) LENGTH: 678 base pairs
(B) TYPE: nucleic acid
(C) STRANDEDNESS: double
(D) TOPOLOGY: linear

(ii) MOLECULE TYPE: cDNA

(iii) HYPOTHETICAL: NO

(iv) ANTI-SENSE: NO

(vi) ORIGINAL SOURCE:
(F) TISSUE TYPE: human placenta

(xi) SEQUENCE DESCRIPTION: SEQ ID NO: 1:

TACGGGCAGA ACTGTCACAA GGGCCCTCCC CACAGCAAAT CTGGAGGTGG GACAGGCGAG 60

GAACCAGGGT CCCAGGGCCT CAATGGGGAG GCAGGACCTG AGGACTCAAC TAGGGAAACT 120

CCTTCACAAG AAAATGGCCC CACAGCCAAG GCCTACACAG GCTTTTCCTC CAACTCGGAA 180

CGTGGGACTG AAGCAGGCCA AGTTGGTGAG CCTGGCATTC CCACTGGTGA GGCTGGGCCT 240

TCCTGCTCTT CAGCCTCTGA CAAGCTGCCT CGAGTTGCTA AGTCCAAGTT CTTTGAGGAC 300

AGTGAGGATG AGTCAGATGA GGCGGAGGAA GAAGAGGAAG ACAGTGAGGA ATGCAGCGAG 360

GAAGAGGATG GCTACAGCAG TGAGGAGGCA GAGAATGAGG AAGATGAGGA TGACACCGAG 420

GAGGCTGAAG AGGACGATGA AGAAGAAGAA GAAGAGATGA TGGTGCCAGG GATGGAAGGC 480

AAAGAGGAGC CTGGCTCTGA CAGTGGTACA ACAGCGGTGG TGGCCCTGAT ACGAGGGAAG 540

CAGTTGATTG TAGCCAACGC AGGAGACTCT CGCTGTGTGG TATCTGAGGC TGGCAAAGCT 600

TTAGACATGT CCTATGATCA CAAACCAGAG GATGAAGTAG AACTAGCACG CATCAAGAAT 660

GCTGGTGGCA AGGTCACC 678

(2) INFORMATION FOR SEQ ID NO: 2:

(i) SEQUENCE CHARACTERISTICS:
(A) LENGTH: 226 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(vi) ORIGINAL SOURCE:
(F) TISSUE TYPE: human placenta

(xi) SEQUENCE DESCRIPTION: SEQ ID NO: 2:

Tyr Gly Gln Asn Cys His Lys Gly Pro Pro His Ser Lys Ser Gly Gly
1               5                   10                  15

Gly Thr Gly Glu Glu Pro Gly Ser Gln Gly Leu Asn Gly Glu Ala Gly
            20                  25                  30

Pro Glu Asp Ser Thr Arg Glu Thr Pro Ser Gln Glu Asn Gly Pro Thr
        35                  40                  45

Ala Lys Ala Tyr Thr Gly Phe Ser Ser Asn Ser Glu Arg Gly Thr Glu

50                  55                  60

Ala Gly Gln Val Gly Glu Pro Gly Ile Pro Thr Gly Glu Ala Gly Pro
65                  70                  75                  80

Ser Cys Ser Ser Ala Ser Asp Lys Leu Pro Arg Val Ala Lys Ser Lys
                85                  90                  95

Phe Phe Glu Asp Ser Glu Asp Glu Ser Asp Glu Ala Glu Glu Glu Glu
            100                 105                 110

Glu Asp Ser Glu Glu Cys Ser Glu Glu Glu Asp Gly Tyr Ser Ser Glu
        115                 120                 125

Glu Ala Glu Asn Glu Glu Asp Glu Asp Asp Thr Glu Glu Ala Glu Glu
    130                 135                 140

Asp Asp Glu Glu Glu Glu Glu Glu Met Met Val Pro Gly Met Glu Gly
145                 150                 155                 160

Lys Glu Glu Pro Gly Ser Asp Ser Gly Thr Thr Ala Val Val Ala Leu
                165                 170                 175

Ile Arg Gly Lys Gln Leu Ile Val Ala Asn Ala Gly Asp Ser Arg Cys
            180                 185                 190

Val Val Ser Glu Ala Gly Lys Ala Leu Asp Met Ser Tyr Asp His Lys
        195                 200                 205

Pro Glu Asp Glu Val Glu Leu Ala Arg Ile Lys Asn Ala Gly Gly Lys
    210                 215                 220

Val Thr
225

(2) INFORMATION FOR SEQ ID NO: 3:

    (i) SEQUENCE CHARACTERISTICS:
        (A) LENGTH: 1641 base pairs
        (B) TYPE: nucleic acid
        (C) STRANDEDNESS: double
        (D) TOPOLOGY: linear

    (ii) MOLECULE TYPE: cDNA

    (iii) HYPOTHETICAL: NO

    (iv) ANTI-SENSE: NO

    (vi) ORIGINAL SOURCE:
        (F) TISSUE TYPE: human placenta

(xi) SEQUENCE DESCRIPTION: SEQ ID NO: 3:
ATGGGTGCCT ACCTCTCCCA GCCCAACACG GTGAAGTGCT CCGGGGACGG GGTCGGCGCC 60
CCGCGCCTGC CGCTGCCCTA CGGCTTCTCC GCCATGCAAG GCTGGCGCGT CTCCATGGAG 120
GATGCTCACA ACTGTATTCC TGAGCTGGAC AGTGAGACAG CCATGTTTTC TGTCTACGAT 180
GGACATGGAG GGGAGGAAGT TGCCTTGTAC TGTGCCAAAT ATCTTCCTGA TATCATCAAA 240
GATCAGAAGG CCTACAAGGA AGGCAAGCTA CAGAAGGCTT TAGAAGATGC CTTCTTGGCT 300
ATTGACGCCA AATTGACCAC TGAAGAAGTC ATTAAAGAGC TGGCACAGAT TGCAGGGCGA 360
CCCACTGAGG ATGAAGATGA AAAAGAAAAA GTAGCTGATG AAGATGATGT GGACAATGAG 420
GAGGCTGCAC TGCTGCATGA AGAGGCTACC ATGACTATTG AAGAGCTGCT GACACGCTAC 480
GGGCAGAACT GTCACAAGGG CCCTCCCCAC AGCAAATCTG GAGGTGGGAC AGGCGAGGAA 540
CCAGGGTCCC AGGGCCTCAA TGGGGAGGCA GGACCTGAGG ACTCAACTAG GGAAACTCCT 600
TCACAAGAAA ATGGCCCCAC AGCCAAGGCC TACACAGGCT TTTCCTCCAA CTCGGAACGT 660
GGGACTGAGG CAGGCCAAGT TGGTGAGCCT GGCATTCCCA CTGGTGAGGC TGGGCCTTCC 720
TGCTCTTCAG CCTCTGACAA GCTGCCTCGA GTTGCTAAGT CCAAGTTCTT TGAGGACAGT 780
GAGGATGAGT CAGATGAGGC GGAGGAAGAA GAGGAAGACA GTGAGGAATG CAGCGAGGAA 840
GAGGATGGCT ACAGCAGTGA GGAGGCAGAG AATGAGGAAG ATGAGGATGA CACCGAGGAG 900
GCTGAAGAGG ACGATGAAGA AGAAGAAGAA GAGATGATGG TGCCAGGGAT GGAAGGCAAA 960
GAGGAGCCTG GCTCTGACAG TGGTACAACA GCGGTGGTGG CCCTGATACG AGGGAAGCAG 1020
TTGATTGTAG CCAACGCAGG AGACTCTCGC TGTGTGGTAT CTGAGGCTGG CAAAGCTTTA 1080
GACATGTCCT ATGATCACAA ACCAGAGGAT GAAGTAGAAC TAGCACGCAT CAAGAATGCT 1140
GGTGGCAAGG TCACCATGGA TGGGCGAGTC AACGGGGGCC TCAACCTCTC CAGAGCCATT 1200
GGGGACCACT TCTATAAGAG AAACAAGAAC CTGCCACCTG AGGAACAGAT GATTTCAGCC 1260
CTTCCTGACA TCAAGGTGCT GACTCTCACT GACGACCATG AATTCATGGT CATTGCCTGT 1320
GATGGCATCT GGAATGTGAT GAGCAGCCAG GAAGTTGTAG ATTTCATTCA ATCAAAGATC 1380

AGCCAGCGTG ATGAAAATGG GGAGCTTCGG TTATTGTCAT CCATTGTGGA AGAGCTGCTG 1440

GATCAGTGCC TGGCACCAGA CACTTCTGGG GATGGTACAG GGTGTGACAA CATGACCTGC 1500

ATCATCATTT GCTTCAAGCC CCGAAACACA GCAGAGCTCC AGCCAGAGAG TGGCAAGCGA 1560

AAACTAGAGG AGGTGCTCTC TACTGAGGGG GCTGAAGAAA ATGGCAACAG CGACAAGAAG 1620

AAGAAGGCCA AGCGAGACTA G 1641

(2) INFORMATION FOR SEQ ID NO: 4:

(i) SEQUENCE CHARACTERISTICS:
(A) LENGTH: 546 amino acids
(B) TYPE: amino acid
(C) STRANDEDNESS: single
(D) TOPOLOGY: linear

(ii) MOLECULE TYPE: peptide

(iii) HYPOTHETICAL: NO

(iv) ANTI-SENSE: NO

(vi) ORIGINAL SOURCE:
(F) TISSUE TYPE: human placenta

(xi) SEQUENCE DESCRIPTION: SEQ ID NO: 4:

Met Gly Ala Tyr Leu Ser Gln Pro Asn Thr Val Lys Cys Ser Gly Asp
1               5                   10                  15

Gly Val Gly Ala Pro Arg Leu Pro Leu Pro Tyr Gly Phe Ser Ala Met
            20                  25                  30

Gln Gly Trp Arg Val Ser Met Glu Asp Ala His Asn Cys Ile Pro Glu
        35                  40                  45

Leu Asp Ser Glu Thr Ala Met Phe Ser Val Tyr Asp Gly His Gly Gly
    50                  55                  60

Glu Glu Val Ala Leu Tyr Cys Ala Lys Tyr Leu Pro Asp Ile Ile Lys
65                  70                  75                  80

Asp Gln Lys Ala Tyr Lys Glu Gly Lys Leu Gln Lys Ala Leu Glu Asp
                85                  90                  95

Ala Phe Leu Ala Ile Asp Ala Lys Leu Thr Thr Glu Glu Val Ile Lys
            100                 105                 110

## Description

The present invention relates to nucleic acids encoding a novel human protein phosphatase of the protein serine/threonine phosphatase family. In particular, it relates to novel DNA sequences encoding a serine/threonine protein phosphatase, to expression plasmids containing said nucleic acids, to host organisms transformed by said expression plasmids, to the production of said protein by culturing said transformant, to antibodies specifically binding to said phosphatase and to agonists and/or antagonists for said protein. Furthermore, the invention relates to serine or threonine residues and epitopes comprising said residues dephosphorylated by said protein and pharmaceutical compositions comprising said protein or agonists or antagonists thereof for the treatment of diseases influenced by changes in phosphorylation which controls e.g. cell proliferation and/or differentiation, to diagnostic kits and to *in vitro* diagnostic methods for the detection of phosphorylation dependent diseases such as e.g. cancer.

Protein phosphorylation-dephosphorylation is a universal mechanism by which different cellular events are regulated. The serine/threonine-specific phosphatases have been classified into four main types according to their in vitro specificity for selected substrates and sensitivity to activators and inhibitors (Ingebritsen, T.S. and Cohen, P. (1983) Eur. J. Biochem. 182, 255-261). Sequence analysis revealed that they can be classified into two major gene families. The first one includes type 1 (PP1), type 2A (PP2A), and type 2B (PP2B) phosphatases, which share 37 to 59 % sequence identity (Barton, G.J. et al., (1994) Eur. J. Biochem. 220, 225-237) in their catalytic domains and are inhibited by okadaic acid (Bialojan, C., and Takai, A. (1988) Biochem. J. 256, 283-290). The second family, the Mg²⁺-dependent phosphatases, also designated type 2C (PP2C), share little sequence similarity with the first family and are insensitive to okadaic acid. cDNA sequences of PP2C α and β from mammalian sources showed > 90 % identity. PP2Cs have been implicated in the regulation of fatty acid and cholesterol biosynthesis (Moore, F. et al. (1991) Eur. J. Biochem. 199, 691-697) and heat shock response (Maeda et al. (1993) Mol. Cell. Biol. 113, 5408-5417, Shiozaki, K. et al. (1994) Mol. Cell. Biol. 14, 3742-375).

The technical problem underlying the present invention is to provide a new human PP2C-like protein phosphatase which is distinct from the other PP2Cs.

The solution to the above technical problem is achieved by providing the embodiments characterized in the claims. Other features and advantages of the invention will be apparent from the description of the preferred embodiments and drawings.

The sequence listings and drawings will now be briefly described.
SEQ ID NO. 1 shows the nucleotide sequence of MP-19, a DNA sequence derived from human placenta.
SEQ ID NO. 2 shows the amino acid sequence of MP-19 as deduced from SEQ ID NO. 1.
SEQ ID NO. 3 shows the nucleotid sequence of MP-19 full-lenght cDNA derived from human placenta.
SEQ ID NO. 4 shows the amino acid sequence of MP-19 as deduced from SEQ ID NO. 3.

The figures show:
Figure 1 shows the alignment of the amino acid sequence of MP-19 with some related proteins of the PP2C family. The asterisk (*) indicates the position of identical amino acids of the compared amino acid sequences.
   - MP19-PCR:: amino acid sequence of MP-19 (SEQ ID NO. 2)
   - PP2C-Human:: human protein phosphatase 2C alpha (Accession No. S87759)
   - PP2C-Rabbit:: rabbit protein phosphatase 2C alpha (Accession No. S87757)
   - PP2C-Rat:: rat protein phosphatase 2C (Accession No. J04503)
Figure 2 is a human RNA master blot. The RNA-dot blot analysis shows hybridization of MP-19 PCR probe with different human RNA samples.
   A1 whole brain, A2 amygdala, A3 caudate nucleus, A4 cerebellum, A5 cerebral cortex, A6 frontal lobe, A7 hippocampus, A8 medulla oblongata, B1 occipital lobe, B2 putamen, B3 substantia nigra, B4 temporal lobe, B5 thalamus, B6 subthalamic nucleus, B7 spinal cord, B8 blank, C1 heart, C2 aorta, C3 skeletal muscle, C4 colon, C5 bladder, C6 uterus, C7 prostate, C8 stomach, D1 tests, D2 ovary, D3 pancreas, D4 pituitary gland, D5 adrenal gland, D6 thyroid gland, D7 salivary gland, D8 mammary gland, E1 kidney, E2 liver, E3 small intestine, E4 spleen, E5 thymus, E6 peripheral leukocyte, E7 lymph node, E8 bone marrow, F1 appendix, F2 lung, F3 trachea, F4 placenta, F5-F8 blank, G1 fetal brain, G2 fetal heart, G3 fetal kidney, G4 fetal liver, G5 fetal spleen, G6 fetal thymus, G7 fetal lung, G8 blank, H1 yeast total RNA, H2 yeast tRNA, H3 *E*. *coli* rRNA, H4 *E*. *coli* DNA, H5 poly r(A), H6-H8 human DNA.

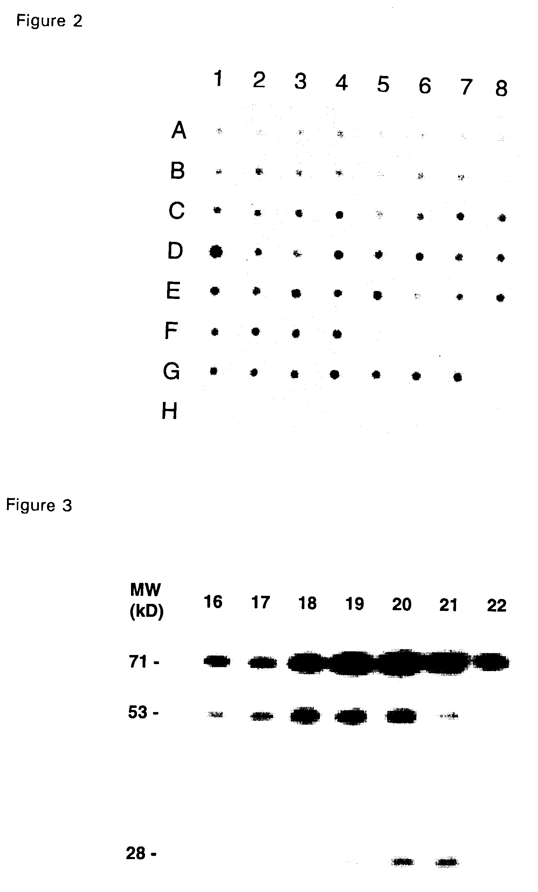

Figure 3 shows the detection of MP-19 after IMAC. The Western analysis was performed after purification of MP-19 using Immobilized Metal Ion Affinity Chromatography (IMAC). Positive signals were obtained from fraction 16 to fraction 22.

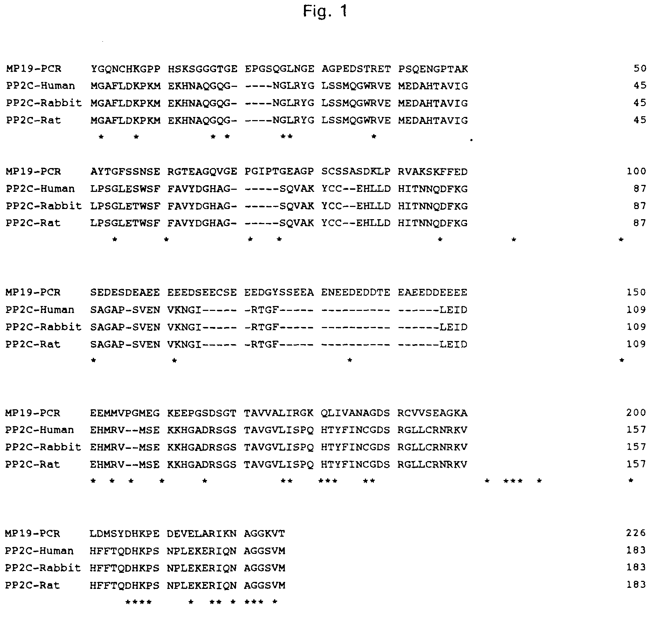

The amino acid sequence alignment of MP-19 with sequences of different PP2Cs (shown in Figure 1) demonstrates the homology of MP-19 to the PP2C family but implicate also that MP-19 belongs to a new protein phosphatase group. The homology of the derived MP-19 amino acid sequence (aa 1 - aa 226) to PP2C from human, rabbit and rat displays a sequence homology of 21.2 %. Moreover a partial sequence of MP-19 (amino acid sequence 158 - 226) which is in the shown alignment not disrupted by gaps indicates a sequence homology of 39.1 % to the compared PP2Cs.

The present invention relates particularly to a novel serine/threonine protein phosphatase and, preferably, provides DNA sequences contained in the corresponding gene.
Such sequences include nucleotide sequences as illustrated in SEQ ID NO. 1 and SEQ ID NO. 3, allelic derivatives of said sequences and DNA sequences degenerated as a result of the genetic code for said sequences. It also includes DNA sequences hybridizing under stringent conditions with the DNA sequence mentioned above. It further includes antisense nucleic acid, preferably antisense MP-19 nucleic acid, directed to the above defined nucleic acid. The terms "nucleic acid sequence" and "nucleotide sequence" refers to DNA or RNA or heterooligomeric sequences, which may be double- or single-stranded.

Although said allelic, degenerate and hybridizing sequences may have structural divergences due to naturally occurring mutations, such as small deletions or substitutions, they will usually still exhibit essentially the same useful properties, allowing their use in basically the same medical or diagnostic applications.

According to the present invention, the term "hybridization" means conventional hybridization conditions, preferably conditions with a salt concentration of 6 x SSC at 62°C to 66°C followed by a one-hour wash with 0.6 x SSC, 0.1% SDS at 62°C to 66°C.

Important biological embodiments of the present invention are DNA sequences of the above and obtainable from vertebrates, preferably mammals such as pig and from rodents such as rat, and in particular from primates such as humans.

Particularly preferred embodiments of the present invention are the DNA sequence termed MP-19 which are shown in SEQ ID NO. 1 and SEQ ID No. 3. The corresponding transcripts of MP-19 were obtained from human placenta tissue and code for a protein showing considerable amino acid homology to the PP2C proteins (shown in Figure 1 deduced from SEQ ID NO. 1). The protein sequence of rabbit and human PP2C α and rat and rabbit PP2C α are described in Mann et al. (1992) Biochim. Biophys. Acta 1130, 100-104. Some typical sequence homologies, which are specific for the known PP2Cs, were also found in the MP-19 sequence. In the present invention, cloning was carried out according to the method described below. Once the DNA sequence has been cloned, the preparation of host cells capable of producing the PP2C-like protein MP-19 and the production of said protein can be easily accomplished using known recombinant DNA techniques comprising constructing the expression plasmids encoding said protein and transforming a host cell with said expression plasmid, cultivating the transformant in a suitable culture medium, and recovering the product having PP2C-like activity.

Thus, the invention also relates to recombinant molecules comprising DNA sequences as described above, optionally linked to an expression control sequence. Such vectors may be useful in the production of the PP2C-like protein in stable or transiently transformed cells. Several animal, plant, fungal and bacterial systems may be employed for the transformation and subsequent cultivation process. Preferably, expression vectors which can be used in the invention contain sequences necessary for the replication in the host cell and are autonomously replicable. It is also preferable to use vectors containing selectable marker genes which can be easily selected for transformed cells. The necessary operation is well-known to those skilled in the art.

It is another object of the invention to provide a host cell transformed by an expression plasmid of the invention and capable of producing a protein of the serine/threonine phosphatase family. Examples of suitable host cells include various eucaryotic and procaryotic cells, such as *E*. *coli*, insect cells, plant cells, mammalian cells, and fungi such as yeast.

Another object of the present invention is to provide a PP2C-like protein or a biologically active fragment thereof encoded by the sequences described above and displaying biological features such as dependency of Mg²⁺ (or Mn²⁺) for activity. Furthermore, the phosphatase catalyzes dephosphorylation of phosphoseryl/threonyl residues of proteins and peptides phosphorylated by cAMP-dependent protein kinases and protein kinase C. It is insensitive to inhibitors like okadaic acid and calyculin A, heparin and PP1 inhibitors 1 and 2. It does not attack phosphorylase α. It is inhibited by polycations and F⁻ ions. A preferred substrate for the PP2C-like protein is the SET protein, suggesting capacities possible relevant to therapeutically treatment of leukemia. Furthermore, the PP2C-like protein prefers basic substrates such as histones, and MBP phosphorylated by cAMP-dependent protein kinase, suggesting a special function for this phosphatase in the brain. The amino acid sequences of especially preferred PP2C-like proteins (MP-19) are shown in SEQ ID NO. 2 and SEQ ID NO. 4.

It is a further aspect of the invention to provide a process for the production of PP2C-like proteins. Such a process comprises cultivating a host cell being transformed with a nucleic acid sequence of the present invention in a suitable culture medium and purifying the PP2C-like protein produced. Thus, this process allows the production of the sufficient amount of the desired protein for use in medical treatments. The host cell is obtainable from bacteria such as *Bacillus spec*. or *Escherichia coli*, from fungi such as yeast, from plants such as tobacco, potato, or *Arabidopsis*, and from animals, in particular vertebrate cell lines such as the Mo-, COS- or CHO cell line.

It is another object of the present invention to provide pharmaceutical compositions containing a therapeutically-effective amount of a PP2C-like protein of the present invention and, optionally, a pharmaceutically acceptable carrier and/or diluent, and/or agonists and/or antagonists thereof. Such a therapeutic composition can be used for the treatment of cancer such as leukemia, brain cancer, breast cancer and prostate cancer. The pharmaceutical composition according to the present invention can also be therapeutically applied for degenerative disorders of the CNS, e.g. Alzheimer's disease, Huntingdon's disease, Parkinson's disease, and epilepsy, and disorders of the reproductive system e.g. fertility disorders or testicular cancer. Another possible clinical application of a PP2C-like protein is the use for treatment of liver diseases, diabetes, and cystic fibrosis. The pharmaceutical composition comprising the protein of the present invention can also be used for treatment of microbial or viral infections. Another application of the pharmaceutical composition of the present invention is the usage of the protein in the regulation of spermatogenesis or the maturation of mammalian germ cells, e.g. for centraception.

Furthermore, the application of the composition is not limited to humans but can include animals, in particular domestic animals, as well.

Finally, another object of the present invention is an antibody or antibody fragment, which is capable of specifically binding to the proteins of the present invention. Methods to raise such specific antibody are known in the art. Such an antibody is preferably a monoclonal antibody. Such antibodies or antibody fragments might be useful for diagnostic methods.

The following examples illustrate in more detail the present invention, but should not be construed as limiting the invention.

### Example 1

### Isolation of MP-19

For the reverse transcription reaction, 5 µg total RNA (0.5 µg/µl) derived from human placenta tissue was heated for 5 minutes and cooled rapidly on ice for 5 minutes. The reverse transcription reagent mixture containing 5 µg total RNA, 38 u of RNA-guard (Pharmacia), 2.5 µg oligomer d(T)12-18 (Boehringer Mannheim), 5x reaction buffer (250 mM Tris/HCl pH 8.5; 50 mM MgCl₂; 50 mM DTT; 600 mM KCl), 10 mM of each dNTP (Pharmacia), 37.5 u of avian myoblastosis virus reverse transcriptase (AMV, Boehringer Mannheim). The reaction mixture (20 µl) was incubated for 90 minutes at 42°C. The resulting placenta cDNA pool was stored at -20°C.

For the primary polymerase chain reaction (PCR), a placenta-derived cDNA pool was used as template in a 50 µl reaction mixture. The PCR reaction was carried out in a RoboCycler Gradient 96 (Stratagene). The amplification was performed in 1x PCR-buffer (10 mM Tris/HCl pH 8.3; 50 mM KCl; 0.001 % gelatine), 1 mM of each dNTP (Pharmacia), 100 pmol of each oligonucleotide (ALK6-N2, 5' - TT(CT)(AG)C(AGCT)AT(AGCT)ATAGAAGAAGATGA - 3' and ALK6-R2, 5' - CC(AGCT)CGCCA(CT)TT(AGCT)CCCATCCA - 3') and 1.5 u Taq polymerase (Perkin Elmer). The PCR reaction contained cDNA corresponding to 30 ng of total RNA as starting material. The reaction mixture was overlaid by 40 µl paraffin incubated for 180s/94°C and subjected to 30 cycles (50s/94°C, 90s/48 °C, 60s/72 °C) with an additional extension for 480s/72 °C in the Thermocycler.

A second round of amplification was performed as described above with exception that 5 µl from the first PCR reaction was used as template DNA for the PCR. A 10 µl sample from the second PCR amplification was fractionated by electrophoresis using a 2 % agarose gel in TBE buffer. After electrophoresis amplified DNA corresponding to a molecular weight of about 600-800 bp was excised from the gel and isolated by 3x freeze/thaw cycles (-20 °C/ + 37 °C) and using the DNA Purification Kit "Easy Pure" (Biozyme, Cat. no. 39001) following the instructions of the manufacturer.

The eluted DNA was amplified a third time as described in the primary PCR with exception that 3 µl of the eluted DNA, resulted from the second round of amplification, was used as template for PCR and the annealing temperature was 56 °C instead of 48 °C. After electrophoresis using a 2% agarose gel in TBE buffer, a distinct DNA band that corresponds to a molecular weight of about 700 bp was eluted with the extraction method described before. After than an additionally purification using the QIAquick 8 PCR Purification Kit (Qiagen, Cat. no. 28144) following the instructions of the manufacturer, was carried out.

Cloning of the purified DNA was established using the Original TA Cloning Kit (Invitrogen, Cat. no. K2000-40). Plasmid DNA from positive clones was isolated with the QIAwell 8 Plus Plasmid Kit (Qiagen, Cat. no. 16142) and sequenced with an automatic DNA sequencer (ALFexpress, Pharmacia). The resulting DNA sequence was analyzed by a homology search with the blast program.

### Example 2

### Isolation of MP-19 full-length cDNA

Isolation of a full-length cDNA clone of MP-19 was performed with a commercial available Human Placenta Lambda cDNA Library (Stratagene, Cat. no. 937225). For screening, a labeled PCR probe was generated from MP-19 DNA (SEQ ID NO. 1). The amplification was performed in 1x PCR-buffer (Qiagen, Germany), 1 mM of dATP, 1 mM of dCTP, 1 mM of dGTP, 0.6 mM of dTTP (Pharmacia, Germany), 0.4 mM of Digoxigenin-11-dUTP (Boehringer, Mannheim), 100 pmol of each oligonucleotide PL19-N1 (5'-GGGCAGAACTGTCACAAGGG-3') and PL19-R1 (5'-CATCCATGGTGACCTTGCCACC-3') and 1 u Taq DNA-polymerase (Qiagen). The PCR mix was overlaid by 40 µl paraffin, incubated for 180s/94 °C and subjected to 30 cycles (60s/94 °C, 60s/58 °C, 60s/72 °C) with an additional extension for 180s/72 °C.

Prehybridization of plaque lift filters from cDNA library were done at 58 °C for 4 h in 0.25 M Na₂HPO₄, 7 % SDS, 1 % BSA, 1 mM EDTA, pH 7.2. Hybridization was carried out with 50 ng labeled MP19 PCR probe for 15 h under same buffer conditions as prehybridization was done. Filter washed 3 times (5 min, 10 min and 15 min) with 30 mM Na₂HPO₄, 0,1 % SDS at 60 °C. Detection of signals were performed with DIG Luminescent detection kit from Boehringer, Mannheim (Cat. no. 1363514). Proceed from a positive signal, clone 39-1 was isolated and sequenced. The resulting DNA-sequence from clone 39-1 conform to Bp. 387-1641 in SEQ ID NO. 3. To generate the 5'cDNA end of MP-19 full-lenght cDNA, 1 µg of a Lambda-DNA preparation from Human Placenta Lambda cDNA Library was subjected to PCR. The ampilfication was performed in 1x PCR-buffer (Qiagen), 1 mM of each dNTP (Pharmacia), 100 pmol of each oligonucieotide MP19-E5 (5'-GGATCCATGGGTGCCTACCTCTCCCAGCCC-3') which was derived from an EST sequence (accession no. AA115688) and MP19-3 (5'-GCCTGTGTAGGCCTTGGCTGTGGGGCC-3') and 1 u Taq DNA-polymerase (Qiagen). The reaction was overiaid by 40 µl paraffin incubated for 180s/94 °C and subjected to 30 cycles (60s/94 °C, 60s/74 °C, 60s/72 °C) with an additional extension for 300s/72 °C. The resulting PCR fragment was subcloned in vector pCR 2.1 (Clontech, Germany). Corresponding DNA was digested with restriction endonucleases Bam HI and Stu I. After that MP-19 Bam HI/Stu I fragment was inserted into clone 39-1. The resulted clone was named 28-9 which DNA-sequence present the full-lenght cDNA-sequence of MP-19 as shown in SEQ ID NO. 3. The corresponding amino acid sequence of MP-19 is shown in SEQ ID NO. 4.

### Gene expression of MP-19 in human tissues

Relative expression of the MP-19 gene was determined by Northern blot analyses. A commercial available Human RNA Master Blot (Clontech, Germany, Cat. no. 7770-1, Lot no. 7090716) was hybridized with the digoxigenin labeled MP-19 PCR probe as described in: Isolation of MP-19 full-length cDNA. 50 different human tissues samples were investigated for MP-19 gene expression. Additionally 8 different negative controls from *E*. *coli*, yeast and human genomic DNA were applied.

Main expression of MP-19 was detected in human testis which is shown in figure 2. Lower expression of MP-19 was detected in human pituitary gland, thymus, small intestine and fetal liver. Basal expression of MP-19 was found in all other human RNA samples. No hybridization signals were detected in negative controls.

### Expression of MP-19 cDNA in E. coli

The cDNA of clone 28-9 was subcloned into the expression plasmid pQE-16 (Qiagen, Germany). This cloning strategy constituted an additional tag of 6 histidine residues at the C-terminus of MP-19. pQE-16 was digested with Bam HI and Bgl II. The 5' part of MP-19 was excised from clone 28-9 with Bam HI and Sac I. To constitute a compatible 3'end of MP-19 for cloning into plasmid pQE-16, a PCR was performed with primer MP19N-Sac I (5'-ACAGCAGAGCTCCAGCCAGAG-3') and MP19R-Bgl II (5'-AGATCTGTCTCGCTTGGCCTTCTTCTTC-3') and template DNA of clone 28-9.5' and 3'end of MP-19 DNA was ligated into pQE-16 to establish MP-19 with His-tag, which was expressed in *E*. *coli* strain M15 (Qiagen). For recombinant expression of MP-19, cells were grown in a 5 l fermenter (Bio Console ADI 1035, Applikon, Netherlands) at 37 °C in LB-Medium until an OD₆₀₀ of 2,5 was reached. After induction with 1 mM β-D-thiogalactopyranoside, cells were grown for additional 4 h until OD₆₀₀ of 9,7 was reached. Cells were harvested by centrifugation at 10.000 x g for 30 min, washed once in 500 ml 1 x PBS buffer (30 min at 10.000 x g) and were frozen in aliquots at -80 °C. For preparation of MP-19 protein, 10 g cells were lysed in 100 ml lysis buffer (50 mM NaH₂PO₄, pH 8,0, 300 mM NaCl, 10 mM imidazole, 100 mg lysozyme (Serva, Germany) and 50 u Benzonase (Merck, Germany)) following by sonication 3 times with a ultrasonic processor (UP-200S, Dr. Hielscher GmbH, Germany) for 3 min at 5 kWsec⁻¹ in an ice/water bath. Cell debris was removed by centrifugation for 30 min at 4 °C and 25.000 x g.

### Purification of recombinant MP-19

Recombinant expressed MP-19 was purified using Immobilized Metal Ion Affinity Chromatography (IMAC) and Reverse Phase Liquid Chromatography (RPLC). The chromatographic purification was realized using the ÄKTA-Explorer system (Pharmacia Biotech, Germany). For IMAC, a 1 ml Hi-Trap cheating column (Pharmacia Biotech) is used. Hi-Trap column was activated with 5 column volumes (cv) of 100 mM NiSO₄, afterwards the column washed with 5 cv water to remove unbound Ni²⁺. Column equilibration was performed with 5 cv of lysis buffer (50 mM NaH₂PO₄, 300 mM NaCl, 10 mM Imidazole, pH 8,0). Cell lysat results from 1 g *E*. *coli* cells was loaded onto column. Afterwards the column washed with lysis buffer to remove unbound protein. Protein were eluted using the following gradient program. Step 1: 20 mM imidazole to 300 mM imidzole within 20 minutes, step 2: 300 mM imidazol to 500 mM imidazol within 10 minutes using buffer A (50 mM Tris, 300 mM NaCl, 20 mM Imidzole, pH 6,0) and buffer B (50 mM Tris, 300 mM NaCl, 500 mM Imidazole, pH 6,0). Flow rate of chromatography were 1 ml/min, detection were performed at 280 nm. Fractions were analyzed by immunological detection, shown in Figure 3. For further purification, positive fractions containing MP-19 were pooled and loaded onto a Resource RPC (3ml) column (Pharmacia Biotech). Column was equilibrated with buffer A (0.1 % trifluor acetic acid) and protein eluted with a linear gradient of buffer B (0,1 % TFA-90 % acetonitrile). Flow rate of chromatography were 3 ml/min, detection were performed at 215 nm.

### Immunolonical Detection of MP-19

Immunological detection of recombinant MP-19 was performed by western blotting using a commercial available monoclonal mouse antibody against histidine-tag (Dianova, Germany, Cat. no. dia900) in combination with Western Light chemoluminescent detection system using the goat anti-mouse-AP antibody (Tropix, U.S.A.).

### Activity assay for recombinant MP-19

A Serine/Threonine Phosphatase Assay System (Promega, Germany, Cat. no. V2460) was used to determine enzymatic activity of recombinant MP-19. This assay use a chemically synthesized phosphopeptide RRA(pT)VA which is a functional substrate for MP-19 phosphatase. The amount of free phosphate which is generated by MP-19 enzymatic reaction was measured by the absorbency of a molybdate:malachit green:phosphate complex (Ekman P. and Jager O. (1993), Anal. Biochem. 214, 138-141, Deana A. D. et al. (1990), Biochimica et Biophysica Acta 1051, 199-202). Assays were performed as described by the manufacturer in PPTase-2C buffer (50 mM imidazole, pH 7,2, 0,2 mM EGTA, 5 mM MgCl₂, 0.02 % β-mercaptoethanol, 0,1 mg/ml BSA). To determine background of this assay, clone pQE-16-dhfr (Qiagen, Germany) was used, which is identical to pQE-MP-19 with exception that vector pQE-16 inserted a mouse dhfr gene instead of MP-19 phosphatase gene.

Results of activity assay of MP-19 shown in table 1. MP-19 has a significant activity in a MgCl₂ containing buffer, but no activity in a CaCl₂ containing buffer, which shows the requirement to Mg^{2+.} Inhibitors like okadaic acid (10 µM) shows no significant reduction of MP-19 activity. Control expression of the mouse dhfr gene shows no activity in the Serine/Threonine Phosphatase Assay System.

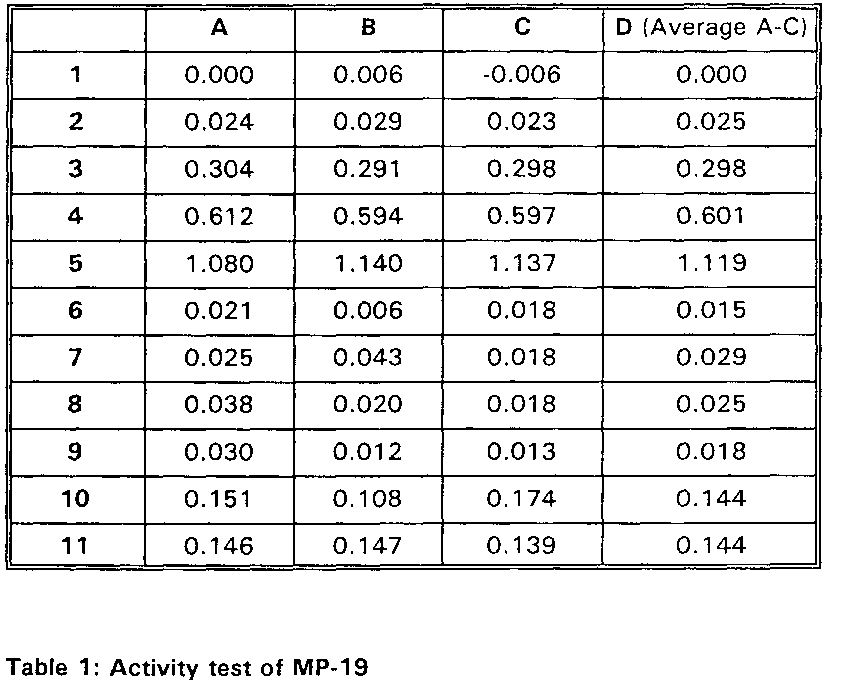

- A1-D1:: Phosphate standard O pmol
- A2-D2:: Phosphate standard 100 pmol
- A3-D3:: Phosphate standard 500 pmol
- A4-D4:: Phosphate standard 1000 pmol
- A5-D5:: Phosphate standard 2000 pmol
- A6-D6:: mouse dhfr gene with substrate (negative control)
- A7-D7:: mouse dhfr gene without substrate (negative control)
- A8-D8:: MP-19 with modified PPTase-2C buffer (5mM MgCl₂ is replaced by 5 mM CaCl₂) and substrate
- A9-D9:: MP-19 with PPTase-2C buffer without substrate
- A10-D10:: MP-19 with PPTase-2C buffer and substrate
- A11-D11:: MP-19 with PPTase-2C buffer, substrate and 10 µM okadaic acid

## Claims

1. A nucleic acid comprising a nucleotide sequence encoding a human serine/threonine phosphatase or a functional fragment thereof that is capable of dephosphorylating serine or threonine residues, wherein the nucleotide sequence comprises:
(a) the nucleotides as shown in SEQ ID NO. 1 or SEQ ID NO. 3; or
(b) a DNA sequence which is degenerate as a result of the genetic code to the DNA sequence of (a); or
(c) an allelic derivative of the sequences of (a) or (b); or
(d) a DNA sequence which is capable of hybridizing to the sequences in (a), (b) and (c), and encoding a protein containing the amino acid sequence as depicted in SEQ ID NO. 2 or SEQ ID NO. 4; or
(e) a nucleotide sequence which is capable of hybridizing to the DNA sequences in (a), (b), (c) and (d), and encoding a protein having essentially the same biological properties as the protein defined in (d).

2. The nucleic acid according to claim 1, wherein the nucleotide sequence is a vertebrate DNA sequence, a mammalian sequence, preferably a primate, human, porcine, or rodent, preferably a rat or rabbit, DNA sequence.

3. A recombinant molecule comprising a nucleic acid according to claim 1 or 2.

4. The recombinant molecule according to claim 3, wherein said nucleic acid sequence is functionally linked to an expression-control sequence.

5. A host containing the nucleic acid according to claim 1 or 2, or the recombinant molecule according to claim 3 or 4.

6. The host according to claim 5, which is a bacterium, a fungus, a plant cell, an animal or a human cell.

7. A process for the production of a PP2C-like protein comprising cultivating a host according to claim 5 or 6 and recovering said PP2C-like protein from the culture.

8. A PP2C-like protein or a biologically active fragment thereof encoded by a nucleic acid according to claim 1 or 2 or by a recombinant molecule according to claim 3 or 4.

9. The protein according to claim 8, comprising the amino acid sequence of SEQ ID NO. 2 or SEQ ID NO. 4.

10. An agonist as a substitute for the protein of claim 8 or 9.

11. An antagonist directed to the protein of claim 8 or 9.

12. A pharmaceutical composition containing the protein or a biologically active fragment thereof according to claim 8 and 9 or the agonist according to claim 10 or the antagonist according to claim 11, and optionally, a pharmaceutically acceptable carrier and/or diluent.

13. The pharmaceutical composition according to claim 12 for the treatment of leukemia, brain cancer, breast cancer, prostate cancer, Alzheimer's disease, Huntingdon's disease, Parkinson's disease, and epilepsy, and of disorders of the reproductive system, or for the regulation of spermatogenesis or the maturation of mammalian germ cells.

14. An antibody or antibody fragment which is capable of specifically binding to the protein of claim 8 and 9 or to the agonist of claim 10 or to the antagonist of claim 11.

15. The antibody according to claim 14, which is a monoclonal antibody.

16. Use of an antibody or antibody fragment according to claim 14 or 15 for detecting the protein or a biologically active fragment thereof as defined in claim 8 or 9.

17. A diagnostic kit containing the agonist according to claim 10 or the antagonist according to claim 11 or the antibody or antibody fragment according to claim 14 or 15.
